# EUROPEAN PATENT SPECIFICATION

(11) **EP 3 865 876 B1**
(45) Date of publication and mention of the grant of the patent: **25.11.2026**
(21) Application number: 19870387.8
(22) Date of filing: 10.10.2019
(51) Int. Cl.: G01N 35/04, G01N 35/10

(54) **ANALYSIS DEVICE**
ANALYSEVORRICHTUNG
DISPOSITIF D'ANALYSE

(30) Priority: 10.10.2018 JP 2018192190; 10.10.2018 JP 2018192191; 10.10.2018 JP 2018192192
(43) Date of publication of application: 18.08.2021
(73) Proprietor: PHC Corporation, Toon-shi Ehime 791-0395 (JP)
(72) Inventor: ISHIGURO, Masatoshi, Ehime 791-0395 (JP); AZUMA, Miyuki, Ehime 791-0395 (JP); OTA, Norihito, Ehime 791-0395 (JP); ABE, Fumitaka, Ehime 791-0395 (JP); KOJIMA, Masami, Ehime 791-0395 (JP); MORIYA, Masamichi, Fujioka-shi, Gunma 375-0043 (JP); SHITARA, Yuichi, Fujioka-shi, Gunma 375-0043 (JP); OHASHI, Yuya, Fujioka-shi, Gunma 375-0043 (JP); HORI, Tetsuya, Fujioka-shi, Gunma 375-0043 (JP)
(74) Representative: Ribeiro, James Michael
(86) International application number: PCT/JP2019/040023
(87) International publication number: WO 2020/075803

(56) References cited:
- EP-A1- 0 549 638
- WO-A1-2006/107016
- WO-A1-2016/136558
- WO-A1-2016/152305
- JP-A- 2001 249 137
- JP-A- H09 145 719
- JP-A- S59 228 167
- JP-Y2- 2 590 688

## Description

### TECHNICAL FIELD

The present invention relates to an analyzer.

### BACKGROUND ART

In the related art, a device has been proposed which can implement a plurality of kinds of analyses with different measurement methods as the biochemical analysis and the immunological analysis (e.g., PTL 1). The device includes (1) a sample supply unit including a sample rack capable of mounting a plurality of biological samples, (2) a first measurement unit capable of holding, detachably and independently of one another, a plurality of mutually independent reaction cuvettes, and including first optical measuring means, (3) sample transport means capable of transporting a biological sample from the sample supply unit to the reaction cuvette on the first measurement unit, (4) a second measurement unit capable of holding, detachably and independently of one another, a plurality of mutually independent reaction cuvettes, and including second optical measuring means, (5) cuvette transfer means capable of transferring the reaction cuvette on the first measurement unit to the second measurement unit, (6) a reagent supply unit including a reagent for use in the measurement at the first measurement unit and the measurement at the second measurement unit, and (7) reagent transport means capable of transporting, independently of one another, reaction reagents from the reagent supply unit to the reaction cuvette on the first measurement unit and/or the second measurement unit. The reaction cuvette on the second measurement unit is transferred from the first measurement unit to the second measurement unit by being carried thereon by the cuvette transfer means after dispensing of the biological sample on the first measurement unit. Then, different measurements can be carried out at the first measurement unit and the second measurement unit.

### PRIOR ART DOCUMENTS

### Patent Document

[Patent Document 1] WO 2006/107016
[Patent Document 2] EP 0549638A1 which discloses an assay or reaction apparatus of modular construction permitting true random operation at the discretion of the user, in particular, an automated multi-test capability assay or reaction apparatus (1) in modular form, comprising a reagent and/or assay sample storage module (2, 3), an incubation module (11), a reaction detection/measurement module (17), and means (16) for independently and as desired both transporting an incubation container (58) between the incubation module (11) and reaction detection/measurement module (17) and for transferring reagent(s) and/or assay sample(s) between different modules without moving reagent(s) and/or assay sample(s) intended for different tests or reactions.

### SUMMARY OF INVENTION

### TECHNICAL PROBLEM

In a case where a plurality of kinds of analyses can be carried out by one device, generally, the device has to be increased in size. Further, in random measurement with a conventional multiple-kind measuring mechanism, it is difficult to carry out a plurality of kinds of measurements while keeping the measurement processing capability (the number of specimen samplings to be carried out per time). For attaining this, the measurement processing capability has to be reduced. Further, the manufacturing cost also has to be undesirably increased due to an increase in size. Under such circumstances, it is an object of the present technology to suppress the increase in size of the device in the analyzer capable of carrying out a plurality of kinds of analyses.

Further, the blood coagulation time varies from specimen to specimen. With a general autoanalyzer, a prescribed measuring time of a specimen is set based on the time required for a large number of coagulation time measurements. When the measurement is not completed within the prescribed measuring time due to uncompletion of the blood coagulation reaction in the prescribed measuring time due to abnormality, or other factors, the process goes to a reanalysis mode, and the measurement is redone from the specimen collection again. However, with this method, a large amount of specimen is required to be prepared, and substantially, the measurement is performed twice. This undesirably results in the reduction of the overall measurement processing efficiency. On the other hand, when the prescribed measuring time is set longer to avoid the reanalysis, the overall measurement processing efficiency is undesirably reduced. Under such circumstances, it is another object of the present invention to improve the measurement processing performances with a device for measuring the degree of progress of a prescribed reaction.

Further, in clinical laboratory examinations, the examination of the blood coagulation ability for examining the clinical condition due to the activity of the blood coagulation factor is the examination generally performed. The physiological hemostases include the coagulation systems of the exogenous type initiated by the combination of the tissue factor (TF) exposed at the injury site and the activating VII-th factor, and of the endogenous type initiated independently of TF. The coagulation test such as the prothrombin time (PT) measurement or the activated partial thromboplastin time (APTT) measurement reproduces this reaction in a test tube, and measures the time required for the thrombin produced by activation of each coagulation factor to convert fibrinogen to fibrin (fibrin formation) (coagulation time) after the addition of a reagent to the test blood plasma. PT is the time until coagulation is observed from addition of lipophilic thromboplastin, and calcium ions with a concentration capable of expressing blood coagulation activity to a test blood plasma, and serves as the comprehensive index of the exogenous type (factors VII, X, and V, prothrombin, and fibrinogen) coagulation activities. APTT is the time until the coagulation is observed from addition of liposome and an activating agent typified by colloidal silica or ellagic acid, and calcium ions with a concentration capable of expressing the blood coagulation activity to a test blood plasma, and serves as the comprehensive index of the endogenous type (factors XII, XI, IX, VIII, X, V, and II, and fibrinogen) coagulation activities.

When the extension of the coagulation time is observed, in order to find out the extension cause, a crossmixing test is carried out. Namely, specimens of a mixture of a patient blood plasma and a normal blood plasma are prepared at a plurality of prescribed ratios, thereby measuring the coagulation time (APTT or PT). With the crossmixing test, for the mixed specimen, after mixing, the result obtained by measuring the coagulation time immediately, and the result obtained by measuring the coagulation time after heating at a prescribed temperature for 2 hours are compared. Further, the crossmixing test can determine whether the extension of the coagulation time is due to the loss of the coagulation factor, due to the inhibitor against the coagulation factor, or due to other factors. However, the crossmixing test requires laborious efforts and time, resulting in a difficulty in automatization. Under such circumstances, it is a still other object of the present invention to provide a technology for supporting the execution of the crossmixing test.

### SOLUTION TO PROBLEM

According to the present invention there is provided an analyzer according to claim 1. The analyzer includes: a first measurement unit for holding a cuvette to which a biological sample is dispensed, and performing a first measurement of a content of the cuvette; a second measurement unit for holding a cuvette, and performing a second measurement different from the first measurement of the content of the cuvette; and a transport unit for gripping the cuvette, and transporting the cuvette to an attachment and detachment position of the first measurement unit or an attachment and detachment position of the second measurement unit. The transport unit moves coaxially along a coaxial guide rail, and the attachment and detachment position of the first measurement unit and the attachment and detachment position of the second measurement unit are provided coaxially substantially in parallel with the guide rail.

By thus making the movement of the transport unit a simple motion along the rail, it is possible to reduce the size of the transport unit, which can suppress the increase in size of the whole device, and also contributes to the reduction of the manufacturing cost.

Further, the analyzer further includes a cuvette supply unit for supplying to a supply port thereof the cuvette. The supply port of the cuvette supply unit, the attachment and detachment position of the first measurement unit, and the attachment and detachment position of the second measurement unit may be arranged coaxially. Further, the analyzer includes a discarding port for discarding a cuvette. The discarding port, the attachment and detachment position of the first measurement unit and the attachment and detachment position of the second measurement unit may be arranged coaxially.

Further, the analyzer further includes: a reagent table for holding a plurality of reagent containers for holding reagents; and a reagent nozzle unit for collecting the reagent from the reagent container at a prescribed collection position, and dispensing the collected reagent in the cuvette held by the first measurement unit or the second measurement unit at a prescribed dispensing position. It is also acceptable that the reagent nozzle unit moves coaxially along the guide rail, and the collection position and the dispensing position are provided coaxially substantially in parallel with the guide rail. With this configuration, the transport unit and the reagent nozzle unit can share the guide rail, which enables the efficient use of the prescribed measuring time and space. Thus, a high measurement processing capability can be achieved, and the whole device can be suppressed from being increased in size.

Further, the analyzer further includes a reagent nozzle washing tank for washing a reagent nozzle arranged at the reagent nozzle unit. It is also acceptable that the reagent nozzle washing tank, the collection position, and the dispensing position are arranged linearly.

The analyzer in accordance with the present invention includes a measurement unit for holding a cuvette to which a biological sample is dispensed, and outputting data indicative of a degree of progress of a prescribed reaction effected in the content of the cuvette, and a processor for extending a measuring time and continuing measurement processing when determination is made that, even after an elapse of a preset measuring time, the prescribed reaction has not been completed on the basis of the data outputted from the measurement unit.

With such a configuration, the measurement time of one specimen is not required to be set longer to be on the safe side, and the reduction of the measurement processing efficiency can be suppressed. Further, if the measurement is terminated temporarily in the measuring time serving as the standard, and the prescribed reaction has not been completed, the measurement of the specimen is redone again. Such processing is inferior in efficiency because redoing takes longer time and because the specimen not having fully undergone the reaction has to be wasted. By adopting the processing in accordance with the present invention, it is possible to eliminate waste of time and waste of specimens, leading to the improvement of the measurement processing efficiency.

The prescribed reaction may be coagulation of blood. Specifically, the reaction is applicable to such analysis.

A mixing test support device as disclosed herein includes: a dispensing mechanism for collecting a biological sample from a container accommodating a biological sample, and dispensing the biological sample to a sample cup; a measurement unit for holding a cuvette accommodating the sample dispensed from the sample cup, and performing measurement of a coagulation time of the content of the cuvette; and a processor for causing the dispensing mechanism to prepare a mixed sample obtained by mixing a normal sample and a sample from a patient, which are accommodated in different containers, in an amount capable of measurements of a plurality of specimens and in a prescribed ratio in the sample cup, and causing the measurement unit to immediately perform measurement of the coagulation time using the prepared mixed sample.

When a prescribed amount of mixed sample for use in the measurement of the coagulation time is prepared by the dispensing mechanism, thereby conducting a mixing test, it is not required of a user to make laborious efforts to prepare the specimens for use in the measurements of the immediate reaction and the delayed reaction. Further, the specimens to be used for the immediate reaction and the delayed reaction, respectively, can be prepared altogether. This eliminates a possibility of an error caused in formation of the specimens. Further, the measurement of the immediate reaction can be started quickly. This eliminates the necessity of making laborious effort of placing an order separately for the measurements of the prepared samples.

Further, the analyzer further includes an input/output device for inputting/outputting information on the basis of an operation by a user. The processor may be configured to cause the measurement unit to perform measurement of a coagulation time by using the mixed sample after heating for a prescribed time, and receive an input of correspondence between the mixed sample which has undergone the measurement of the coagulation time immediately via the input/output device, and the mixed sample which has undergone the measurement of the coagulation time after heating for a prescribed time, acquire information regarding a coagulation time measured for corresponding mixed samples from the measurement unit, and cause the input/output device to output the information. For example, heating is performed at 37 degrees for two hours. Each APTT of the immediate reaction and the delayed reaction can be measured with the present device. For this reason, the results of the immediate reaction and the delayed reaction can be outputted altogether in a form easy for a user to make comparison therebetween.

Incidentally, the contents described in Solution to Problem can be combined as much as possible within the range not deviating from the objects and the technical idea of the present invention. Further, the contents of Solution to Problem can be provided as a system including a device such as a computer or a plurality of devices, the method executed by a computer, or a program to be executed by a computer. The program can also be configured to be executed on a network. Incidentally, it is also acceptable that a recording medium holding the program is provided.

### ADVANTAGEOUS EFFECT OF INVENTION

With an analyzer capable of carrying out a plurality of kinds of analyses, it is possible to suppress the increase in size of the device and the manufacturing cost thereof. Further, it is possible to improve the measurement processing performances.

### BRIEF DESCRIPTION OF DRAWINGS

[Fig. 1]

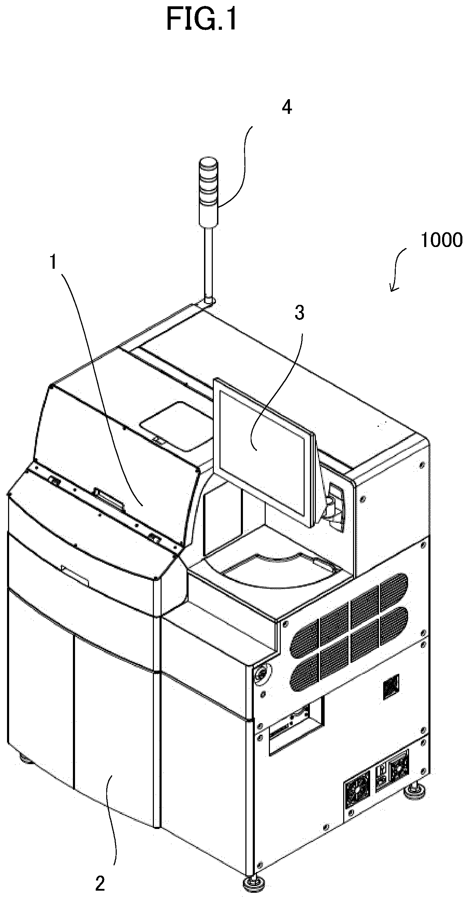

   Fig. 1 is a view showing one example of a composite analyzer.
[Fig. 2]

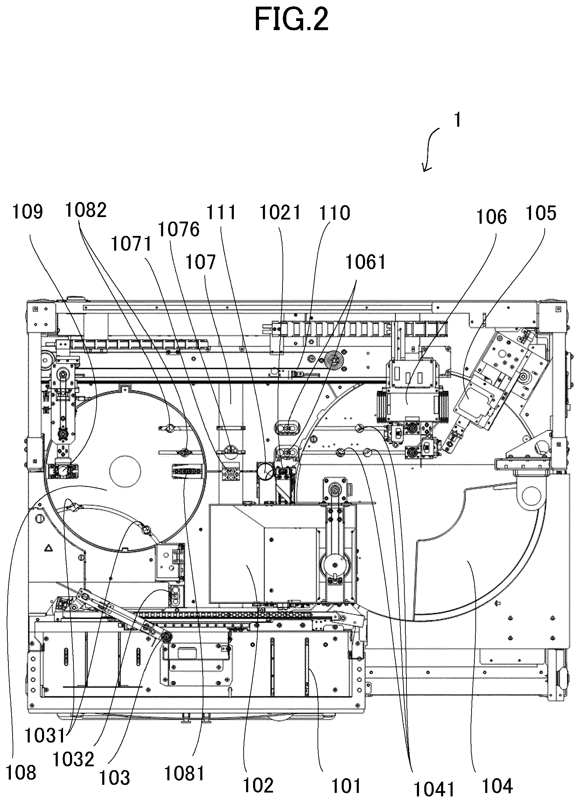

   Fig. 2 is a plan view showing one example of a configuration of the inside of a measurement unit accommodation part of the composite analyzer.
[Fig. 3A]

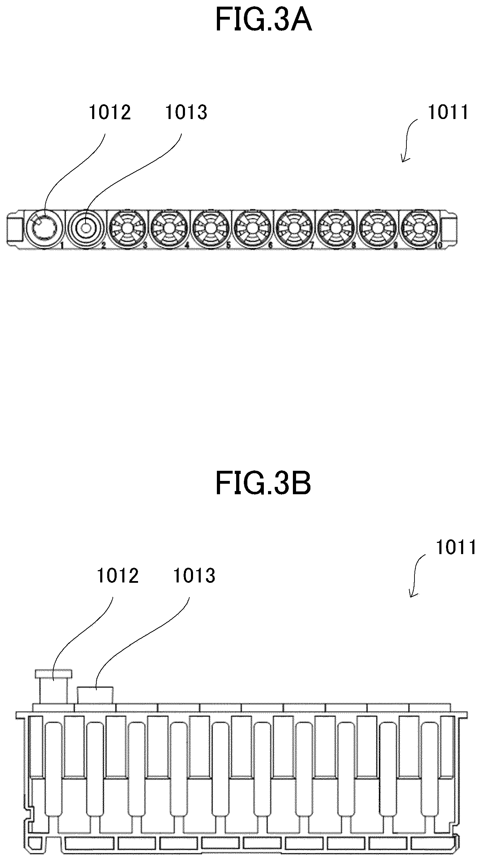

   Fig. 3A is a view showing one example of a sample rack.
[Fig. 3B]
   Fig. 3B is a view showing one example of the sample rack.
[Fig. 4]

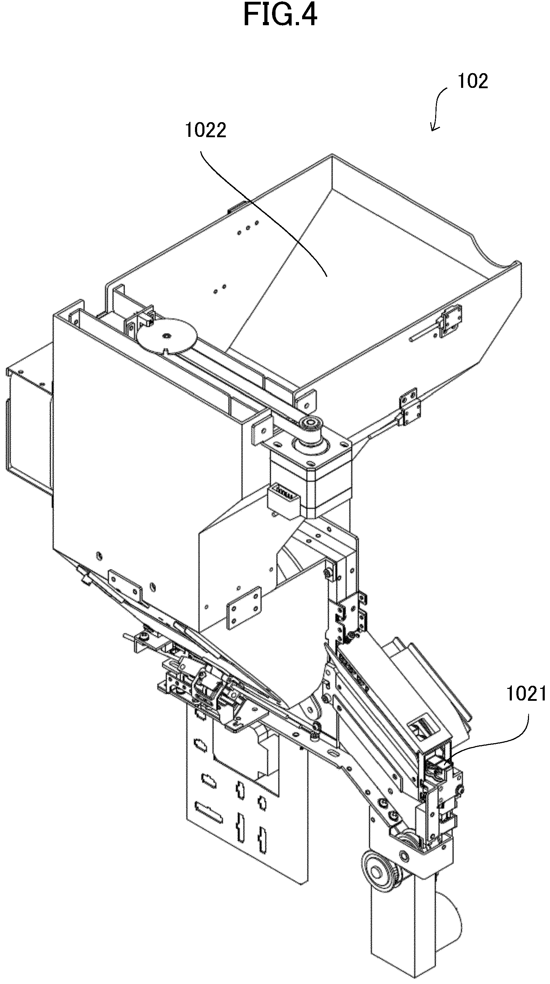

   Fig. 4 is a view showing one example of a cuvette supply unit.
[Fig. 5]

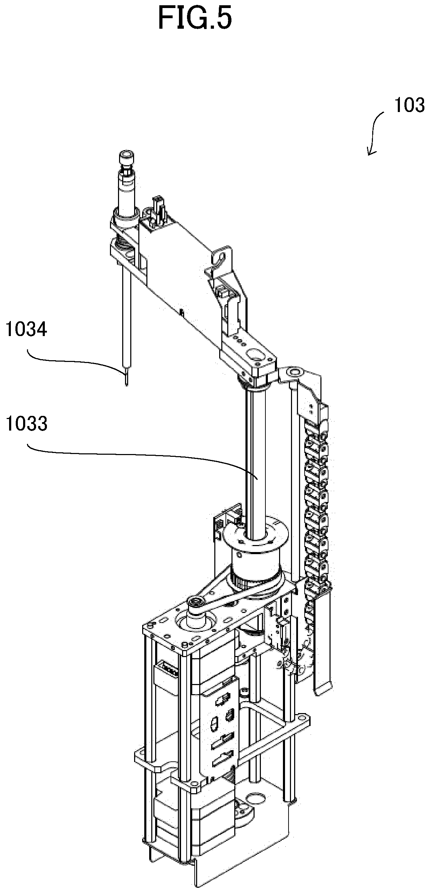

   Fig. 5 is a view showing one example of a sample nozzle unit.
[Fig. 6]

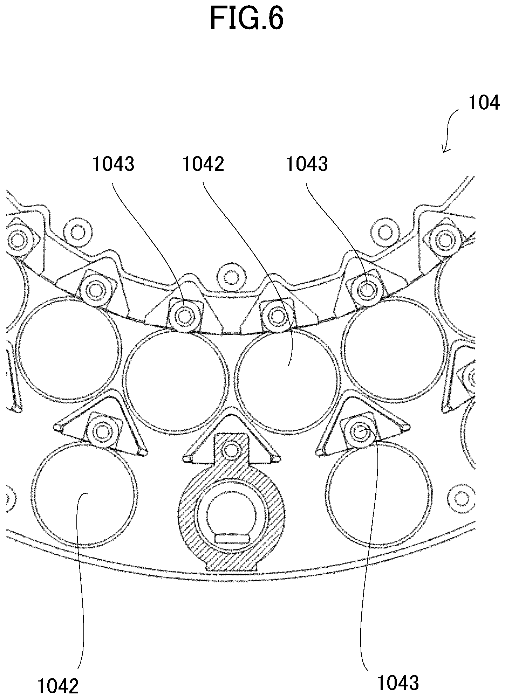

   Fig. 6 is a view showing one example of a partial reagent table.
[Fig. 7]

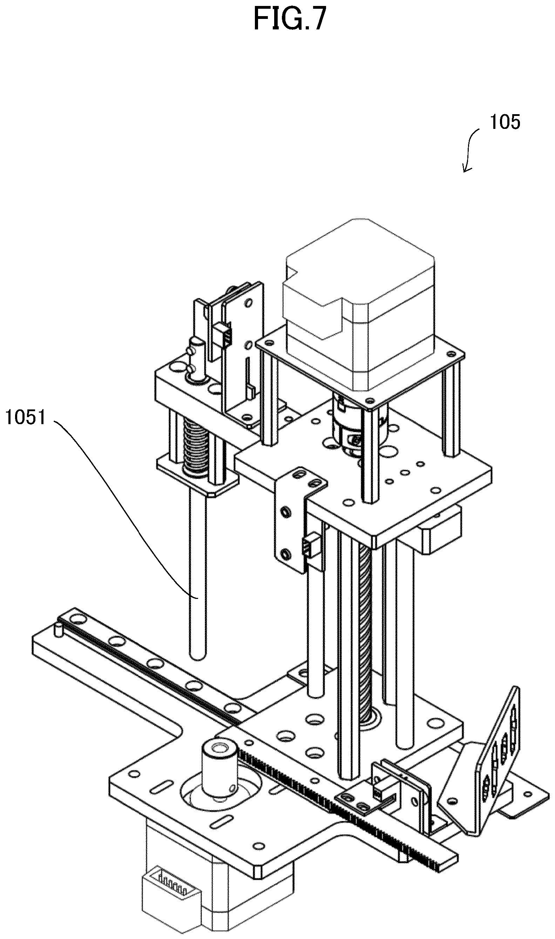

   Fig. 7 is a view showing one example of a reagent lid opening/closing unit.
[Fig. 8]

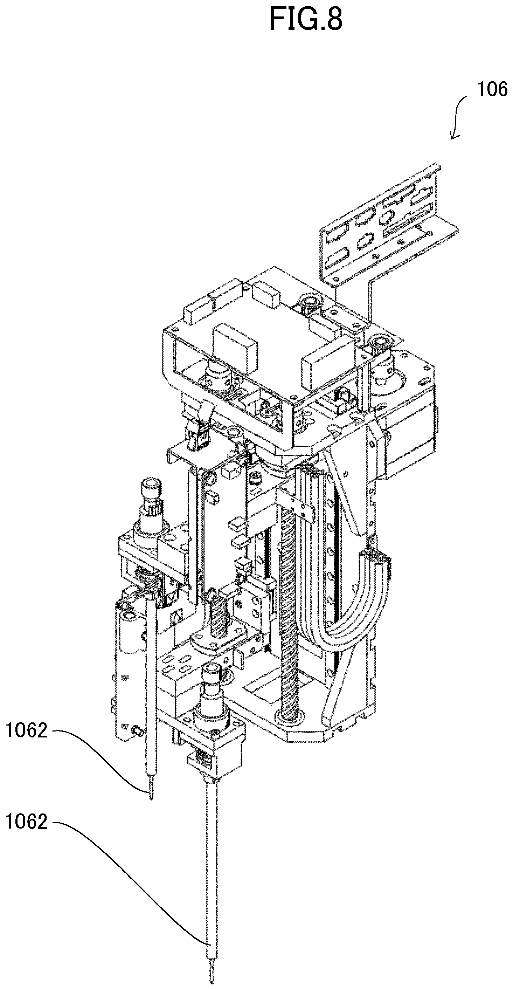

   Fig. 8 is a view showing one example of a reagent nozzle unit.
[Fig. 9]

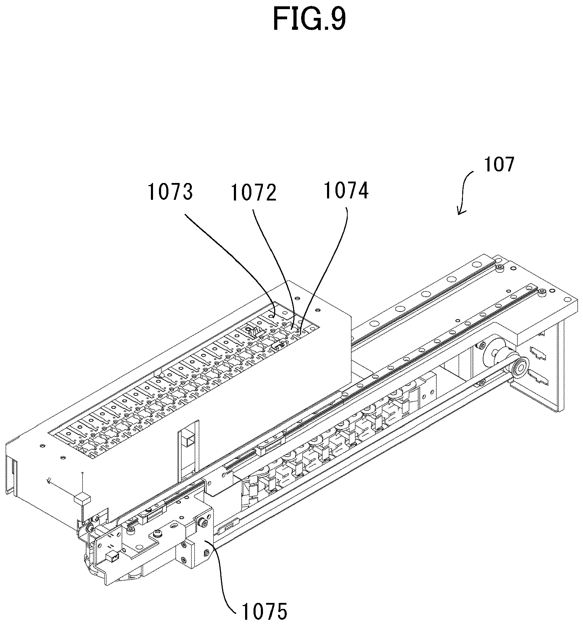

   Fig. 9 is a view showing one example of a coagulation table.
[Fig. 10]

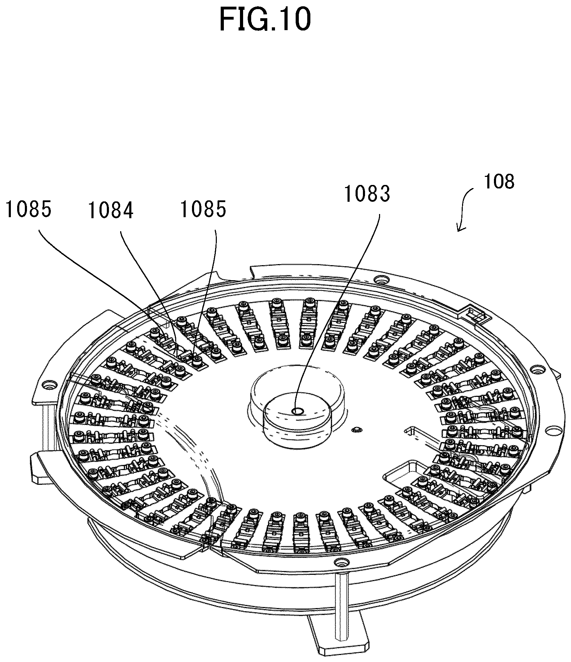

   Fig. 10 is a view showing one example of a LPIA table.
[Fig. 11]

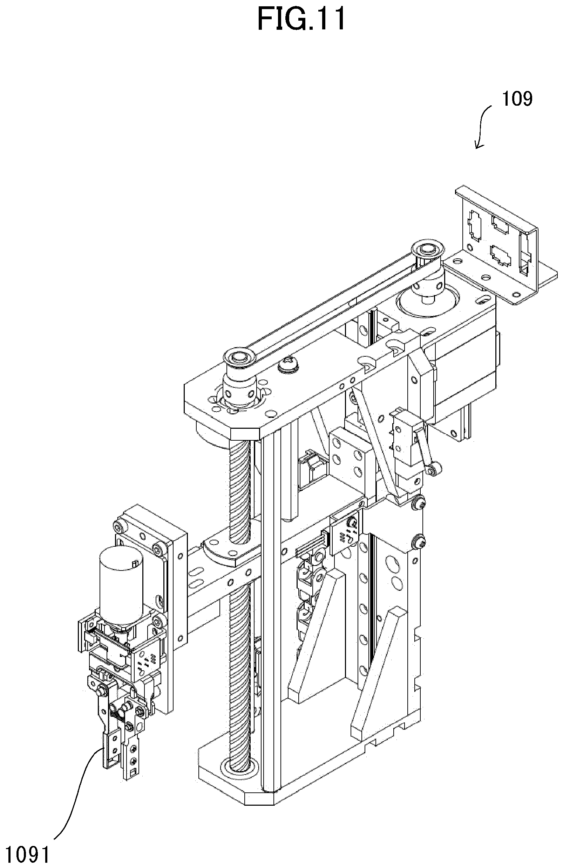

   Fig. 11 is a view showing one example of a cuvette chuck unit.
[Fig. 12A]

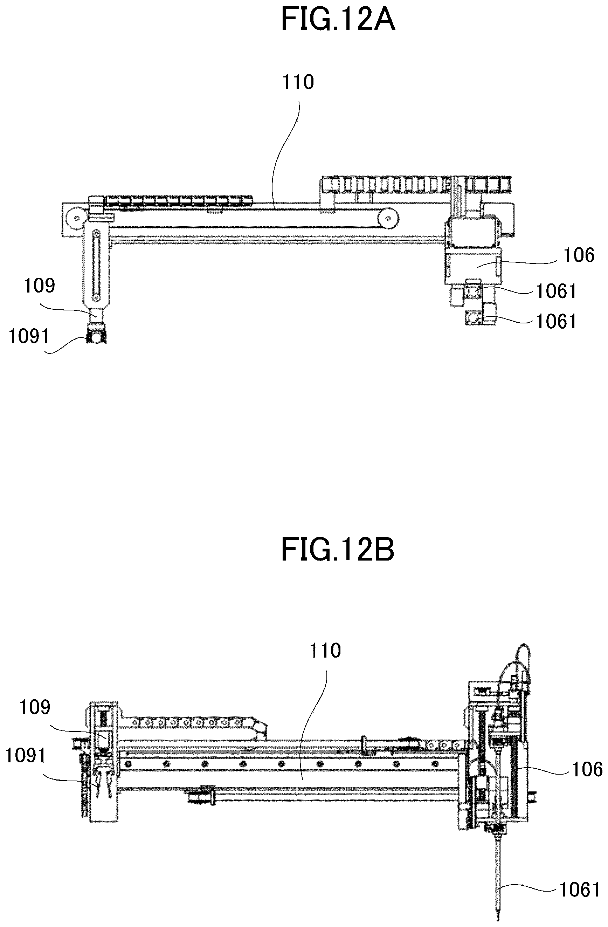

   Fig. 12A is a plan view showing one example of a rail.
[Fig. 12B]
   Fig. 12B is a front view showing one example of the rail.
[Fig. 13]

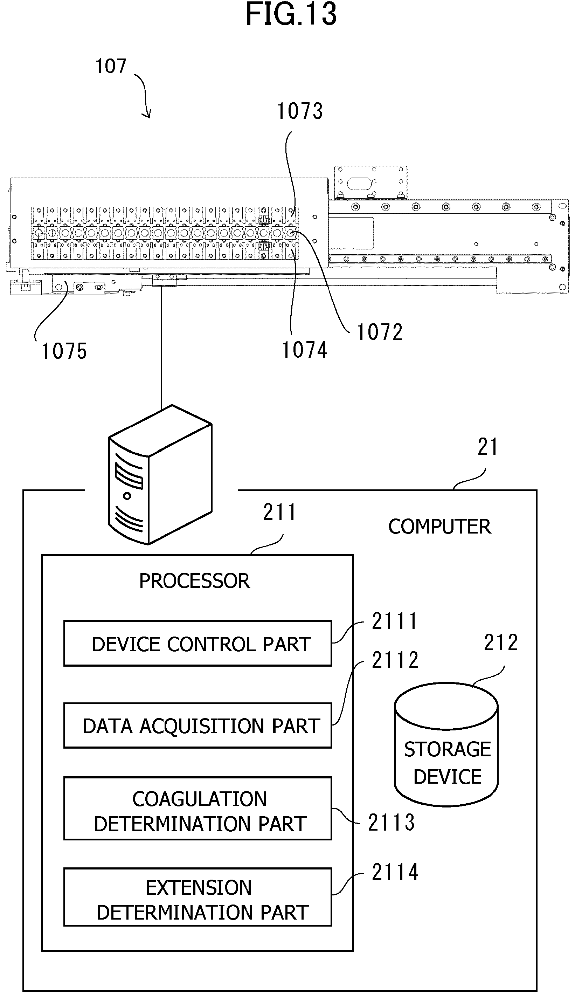

   Fig. 13 is a functional block view showing one example of a computer.
[Fig. 14]

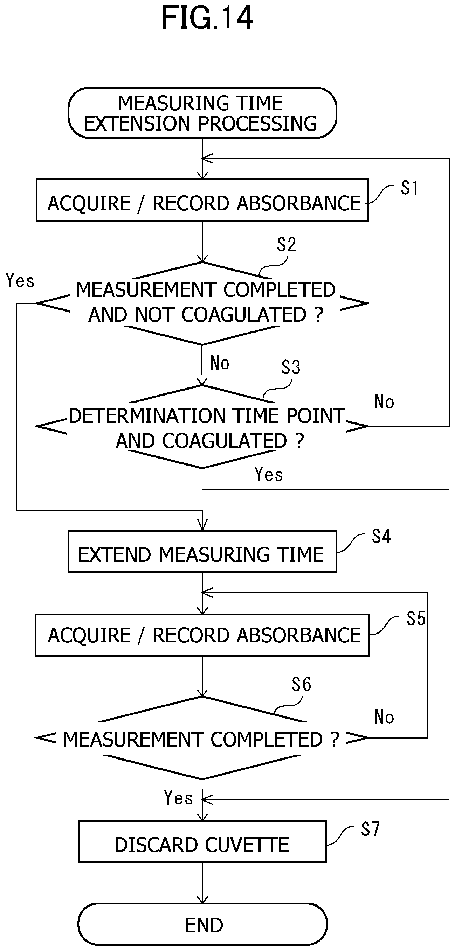

   Fig. 14 is a processing flowchart showing one example of measuring time extension processing.
[Fig. 15]

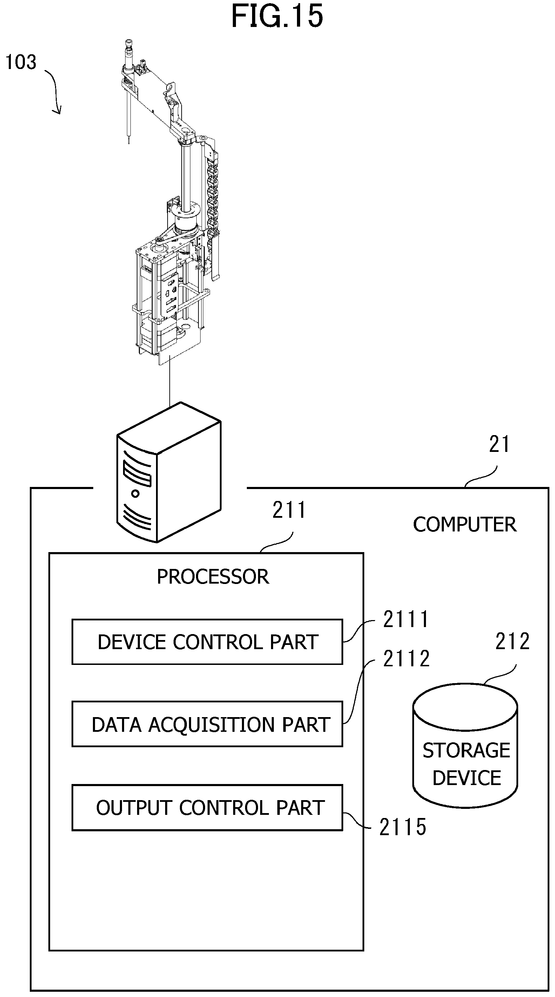

   Fig. 15 is a functional block view showing one example of the computer.
[Fig. 16]

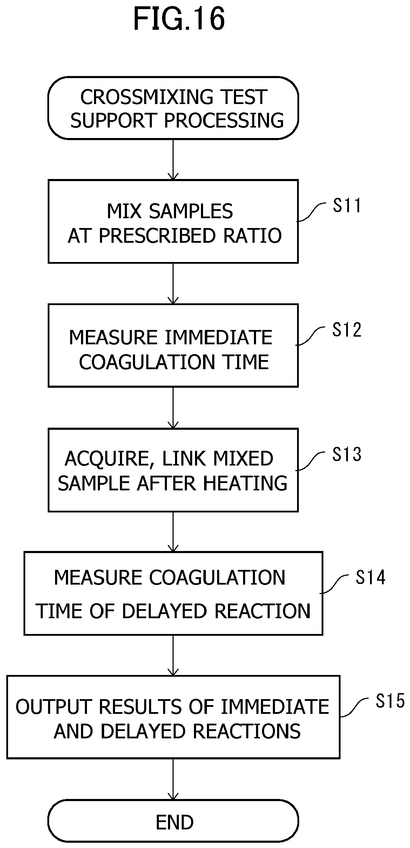

   Fig. 16 is a processing flowchart showing one example of mixing test support processing.
[Fig. 17]

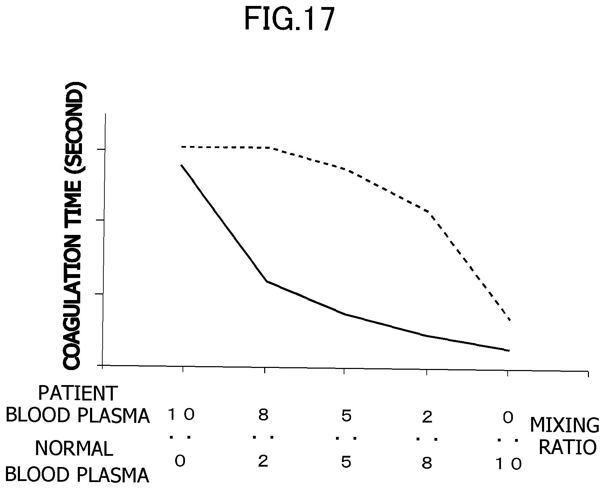

   Fig. 17 is a view showing one example of the outputted measurement results.
[Fig. 18]

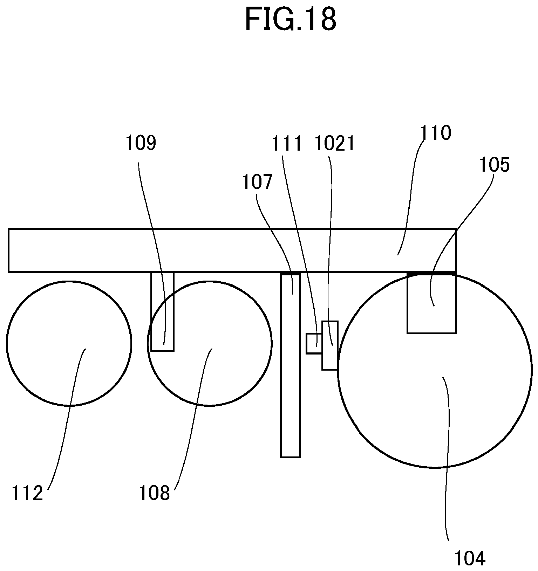

   Fig. 18 is a schematic view showing one example of a composite analyzer including three measurement units.

### DESCRIPTION OF EMBODIMENTS

Below, a composite analyzer in accordance with an embodiment will be described by reference to the accompanying drawings.

### <Device configuration>

Fig. 1 is a view showing one example of the outward appearance of a composite analyzer 1000. The composite analyzer 1000 is an analyzer for carrying out a plurality of kinds of analyses with different precisions such as biochemical analysis and immunological analysis. The composite analyzer 1000 can carry out, for example, LPIA (Latex Photometric Immunoassay) and blood coagulation time measurement. Further, the composite analyzer 1000 includes a measurement unit accommodation part 1, a tank, etc.-accommodating part 2, a monitor 3, and a status output part 4. The measurement unit accommodation part 1 accommodates a plurality of measurement units in accordance with the embodiment, and the like. The tank, etc.-accommodating part 2 accommodates therein tanks for respectively retaining pure water, washing water, and drained water, the discarding box for accumulating cuvettes to be discarded, a computer for controlling the processing performed by the measurement unit accommodation part 1, and the like. The monitor 3 is connected to the computer, and outputs the measurement progress status, the results, and the like. Further, the monitor 3 may be an input/output device capable of undergoing an input operation by a user such as a touch screen. The status output part 4 is connected to the computer, or the like, and turns on and off, or lights up a warning light for notifying a user of abnormal conditions when the abnormal conditions occur in the processing executed by the measurement unit accommodation part 1.

Fig. 2 is a plan view showing one example of the configuration of the inside of the measurement unit accommodation part 1 of the composite analyzer 1000. The measurement unit accommodation part 1 has a transport space 101 of a sample rack, a cuvette supply unit 102, a sample nozzle unit 103, a reagent table 104, a reagent lid opening/closing unit 105, a reagent nozzle unit 106, a coagulation table 107, a LPIA table 108, a cuvette chuck unit 109, a rail 110, and a cuvette discarding port 111. The transport space 101 includes a sample rack 1011 mounted therein, and is transported on the table by a prescribed mechanism. The sample rack 1011 holds a plurality of blood collection tubes each for accommodating a biological sample such as a blood specimen. The cuvette supply unit 102 supplies cuvettes in a prescribed shape for use at the composite analyzer 1000. Incidentally, the cuvettes are supplied sequentially one by one from a cuvette supply port 1021. The sample nozzle unit 103 is a unit having a nozzle connected to a pump, and moving within a prescribed movable range based on the control by the computer, thereby collecting a sample from the blood collection tube, and discharging the sample to the cuvette of the LPIA table 108. The reagent table 104 is a disk-shaped holding part holding a plurality of reagent bottles for accommodating a reagent therein, and rotating based on the control by the computer. The held reagent bottle is collected at the reagent nozzle unit 106 at a prescribed collection position 1041. The reagent lid opening/closing unit 105 is a unit moving within a prescribed movable range based on the control by the computer, thereby opening/closing the lid of the reagent bottle. The reagent nozzle unit 106 is a unit having a nozzle connected to a pump, and moving within a prescribed operation range based on the control by the computer, thereby collecting a reagent from the reagent bottle, and discharging the reagent to the cuvette. The coagulation table 107 is a holding part including a plurality of holes for arranging and holding a plurality of cuvettes for measuring the degree of coagulation of the contents of the cuvettes. Incidentally, a light source and a light receptive part are arranged across the held cuvette, so that the degree of coagulation is measured based on the absorbance or the transmittance of the contents. The LPIA table 108 is a disk-shaped holding part for arranging and holding a plurality of cuvettes in a circular shape in a plan view, and rotating based on the control by the computer for measuring the antigen amount in the specimen by LPIA. The held cuvette is attached and detached by the cuvette chuck unit 109 at a prescribed attachment and detachment position 1081, and a reagent is dispensed at a prescribed dispensing position 1082. The cuvette chuck unit 109 moves within a prescribed movable range based on the control by the computer, and holds and moves the cuvette. The rail 110 is a linear rail. The reagent nozzle unit 106 and the cuvette chuck unit 109 are respectively connected to the rail 110, and can move in roughly parallel with the rail 110 along the direction of extension of the rail 110. The cuvette discarding port 111 is an opening communicating with the discarding box housed in the tank, etc.-accommodating part 2, and can discard the cuvettes, and the like in the cuvette discarding port 111.

Figs. 3A and 3B are each a view showing one example of the sample rack. A sample rack 1011 includes a plurality of holders for holding a blood collection tube 1012 for accommodating a sample. Further, the sample rack 1011 is transported based on the control by the computer, and can arrange a desirable blood collection tube 1012 at a prescribed collection position. The collection position is present on the orbit on which the sample nozzle unit 103 moves in a circular arc shape in a plan view, and the sample is dispensed to the cuvette held in the holding hole of the LPIA table 108 by the sample nozzle unit 103. Incidentally, the composite analyzer 1000 may be configured by including a reading device for optically reading the identifying information such as a bar code or a two dimensional code attached on the label of the blood collection tube 1012, thereby allowing the identification of a desirable blood collection tube. Further, it may be configured such that a sample cup is arranged on the blood collection tube, thereby allowing preparation of a diluted sample, a mixed sample, or the like in the sample cup.

Fig. 4 is a view showing one example of the cuvette supply unit. The cuvette supply unit 102 supplies cuvettes to be charged into a hopper 1022 from the cuvette supply port 1021 of the end of the slope-shaped exit one by one in a prescribed direction by a prescribed mechanism.

Fig. 5 is a view showing one example of the sample nozzle unit. For the sample nozzle unit 103, a nozzle 1034 moves drawing a circular arc-shaped orbit in a plan view about a prescribed rotation shaft 1033 as the center. Then, the sample nozzle unit 103 dispenses the sample from the blood collection tube 1012 of the sample rack 1011 moved to the collection position to the cuvette of the LPIA table 108 moved to the dispensing position 1031. The sample nozzle unit 103 is also referred to as the "dispensing mechanism" in accordance with the present invention.

Fig. 6 is a plan view showing one example of the partial reagent table. The reagent is, for example, but is not limited to latex, or a coagulation time reagent. The configuration of the reagent table can be appropriately changed according to the kind of the measurement principle. The reagent table 104 is a disc-shaped table rotating about the prescribed rotation shaft as the center. The table is provided with a plurality of setting parts 1042 each for holding a reagent bottle in a ring shape. Incidentally, in the present embodiment, the setting parts 1042 are disposed in a double ring shape, and the number of the setting parts 1042 and the number of the rings have no particular restriction. Further, the reagent table 104 includes rod-shaped convex parts 1043 protruding vertically upward around the setting part 1042. In the present embodiment, each setting part 1042 is provided with the convex parts 1043 on the rotation shaft side. Further, the reagent bottle in accordance with the present embodiment is in a roughly cylindrical shape, and has an engage part into which the convex part 1043 can be inserted at the side surface thereof. The engage part is a through hole penetrating in a vertical direction or a concave part open vertically downward, and can fix a reagent bottle by inserting the convex part 1043 into the engage part. Further, the table rotates clockwise or counterclockwise based on the control by the computer, and stops at a prescribed position. For example, each setting part 1042 stops at the reagent collection position of the point of intersection with the orbit on which the nozzle of the reagent nozzle unit 106 moves in a plan view, the lid opening/closing position under the reagent lid opening/closing unit 105, the attachment and detachment position for a user to attach/detach a reagent bottle, and the like. Incidentally, the composite analyzer 1000 may include a reading device for optically reading identifying information such as a bar code or a two dimensional code attached on the label of the reagent bottle, thereby allowing identification of a desirable reagent bottle. For example, the reading device is disposed toward the direction of the rotation shaft from the outside of the reagent table 104 in a plan view. Further, the plurality of setting parts 1042 disposed in a double ring shape are arranged such that, for example, the setting parts 1042 on the inner circumference side and the setting parts 1042 on the outer circumference side are set in a staggered arrangement along the circumference. As a result, the reagent bottles do not overlap each other as seen from the reading device, so that rotation of the reagent table 104 allows reading of the labels of all the reagent bottles. Incidentally, the setting parts 1042 may be disposed in three or more concentric circles. When the holding holes are disposed in a plurality of concentric circles, the table may be one disk rotating integrally, or may be formed of a plurality of ring-shaped disks whose plural concentric circles can rotate independently of one another. In the case of the structure in which the plurality of ring-shaped disks rotate independently, individual rings rotate clockwise or counterclockwise independently based on the control by the computer, and stop independently of one another. The kind and the number of the used reagents vary according to the measurement method. By rotating the reagent table 104, the setting part 1042 at which a desirable reagent bottle is arranged can be moved to the collection position 1041, and can be used according to the measurement. Further, when it is configured such that the composite analyzer 1000 reads the identifying information pasted on the reagent bottle, and automatically stores the arrangement of the reagent bottles on the reagent table 104, a user can complete preparation only by setting the reagent bottles necessary for the measurement without caring about the setting place, resulting in an improvement of the convenience. Further, the reagent table 104 includes a plurality of setting parts 1042. For this reason, the measurable items can be increased without replacing reagent bottles, so that the convenience is high.

Fig. 7 is a view showing one example of the reagent lid opening/closing unit. The reagent lid opening/closing unit 105 opens and closes the lid of the reagent bottle at the tip part 1051. The lid of the reagent bottle in accordance with the present embodiment is connected by a hinge, and opens and closes by applying a moment in a prescribed opening/closing direction to the lid. The lid of the reagent bottle is provided with a convex part protruding in a direction roughly perpendicular to the lid, and to be applied with a force by the tip part 1051. When the convex part is applied with a force in a prescribed lid opening direction, a moment rotating the lid in the direction in which the lid is opened with the hinge as the fulcrum acts on the hinge. The tip part 1051 of the reagent lid opening/closing unit 105 moves along the radial direction of the reagent table 104 in a plan view, and is displaced onto the orbit on which the reagent bottles are moved due to the rotation of the reagent table 104, and comes in contact with the convex part protruding from the lid, and opens the lid. Further, the tip part 1051 of the reagent lid opening/closing unit 105 is also displaced in the vertical direction by a prescribed driving mechanism. The tip part 1051 applies a force on the lid in the lid closing direction opposite to the lid opening direction, and presses the lid vertically downward, thereby to be able to close the lid of the reagent bottle.

Fig. 8 is a view showing one example of the reagent nozzle unit. The reagent nozzle unit 106 in accordance with the present embodiment includes two nozzles 1061 for collecting and discharging the reagent. The two nozzles 1061 go upward and downward in the vertical direction independently of each other, and can collect and discharge the reagent. Further, the reagent nozzle unit 106 moves along the rail 110 based on the control by the computer, and collects the reagent from the reagent bottle at the collection position of the reagent bottle on the reagent table 104, and discharges the reagent to the cuvette at the dispensing position of the cuvette on the LPIA table 108. Thus, the reagent nozzle unit 106 can move linearly along the rail 110 between a prescribed position on the reagent table 104 and a prescribed position on the LPIA table 108. Further, in a plan view, the collection position is disposed at the point of intersection of the path on which one nozzle 1061 of the reagent nozzle unit 106 in accordance with the present embodiment moves linearly, and the orbit on the outer circumference side of the cuvettes arranged in a double ring shape of the reagent table 104. Further, the collection position is also disposed at the point of intersection of the path on which the other nozzle 1061 of the two nozzles of the reagent nozzle unit 106 in accordance with the present embodiment linearly moves, and the orbit on the inner circumference side. Further, the dispensing positions are disposed, in a plan view, at the points of intersection of the paths on which the two nozzles of the reagent nozzle unit 106 respectively move linearly, and the orbit for the rotation of the cuvettes arranged in one ring shape at the LPIA table 108, respectively.

Fig. 9 is a view showing one example of the coagulation table. The coagulation table 107 is, for example, a table for mounting cuvettes thereon for carrying out the blood coagulation time measurement. The coagulation table 107 includes a plurality of holding holes 1072 for holding cuvettes linearly in the direction roughly perpendicular to the direction of extension of the rail 110. Further, a light source 1073 is arranged at one side, and a light receptive part 1074 is arranged on the other side across the cuvette held by the holding hole 1072. Then, the degree of coagulation of the contents is measured by the absorbance or the transmittance of a light with a prescribed wavelength of the contents of the cuvette. Further, the coagulation table 107 includes a driving part 1075 for sliding the table in the direction roughly perpendicular to the direction of extension of the rail 110. Then, a desirable holding hole 1072 can be moved to the attachment and detachment position 1071 of the point of intersection with the orbit on which the cuvette chuck unit 109 moves. Further, the cuvette chuck unit 109 can allow the holding hole 1072 to hold the cuvette, or can remove the cuvette from the holding hole 1072 at the prescribed attachment and detachment position 1071. To the holding hole 1072 of the coagulation table 107, the cuvette to which the sample is dispensed at the holding hole of the LPIA table 108 is transported by the cuvette chuck unit 109. Incidentally, the light sources 1073 and the light receptive parts 1074 are disposed as many as the number of the holding holes 1072, and the holding hole 1072, the light source 1073, and the light receptive part 1074 move integrally. Therefore, even during the time during which the table moves, each cuvette can be continued to be measured for the absorbance, and the like.

Fig. 10 is a view showing one example of the LPIA table. The LPIA table 108 is a table for mounting cuvettes thereon, for example, for carrying out the measurement of the antigen amount by the latex coagulation method. The LPIA table 108 is a disk-shaped table rotating about the prescribed rotation shaft 1083 as the center, and is provided with a plurality of holding holes 1084 for holding cuvettes along the circumference. The table rotates clockwise or counterclockwise based on the control by the computer, and stops at a prescribed position. Further, each holding hole 1084 is provided with a spring 1085 for pressing the cuvette.

Fig. 11 is a view showing one example of the cuvette chuck unit. The cuvette chuck unit 109 is a unit including a two-finger gripper 1091 at the tip part, and for gripping and transporting the cuvette. Further, the cuvette chuck unit 109 moves linearly in the horizontal direction along the rail 110, and grips and drops cuvettes at the attachment and detachment positions of the reagent table 104 and the LPIA table 108, the cuvette supply port 1021, the cuvette discarding port 111, and the like.

Fig. 12A is a plan view showing one example of the rail including the cuvette chuck unit and the reagent nozzle unit. Fig. 12B is a front view showing one example of the rail including the cuvette chuck unit and the reagent nozzle unit. The rail 110 is a rail-shaped member connected to the reagent nozzle unit 106 and the cuvette chuck unit 109, and serving as a guide when the reagent nozzle unit 106 or the cuvette chuck unit 109 moves. Namely, with the composite analyzer 1000, the attachment and detachment positions of the reagent table 104, the coagulation table 107, and the LPIA table 108, the cuvette supply port 1021, the cuvette discarding port 111 are arranged on a straight line roughly in parallel with the rail 110. Further, the collection position 1041 of the reagent table 104, the dispensing position 1076 of the reagent of the coagulation table 107, and the dispensing position 1082 of the reagent of the LPIA table 108 are also arranged on a straight line roughly in parallel with the rail 110. Incidentally, the nozzle 1061 of the reagent nozzle unit 106 and the two-finger gripper 1091 of the cuvette chuck unit 109 move upward and downward in the vertical direction. However, the movement in the forward/backward (front or depth) direction thereof is prevented. The reagent nozzle unit 106 and the cuvette chuck unit 109 are connected to the front side of the rail 110, and move using the rail 110 in common. For example, the cuvette chuck unit 109 is retreated to the left side in a plan view and in a front view. As a result, the nozzle 1061 of the reagent nozzle unit 106 can be moved to the 25 the dispensing position 1082 of the LPIA table 108. Further, for example, the reagent nozzle unit 106 is retreated to the right side in a plan view and in a front view. As a result, the two-finger gripper 1091 of the cuvette chuck unit 109 can be retreated to the cuvette supply port 1021 of the cuvette supply unit 102.

Further, the cuvette discarding port 111 is the input port for the cuvette chuck unit 109 to drop cuvettes thereinto for accumulating the cuvettes in the discarding box stored in the tank, etc.-accommodating part **2.** In the tank, etc.-accommodating part 2, a tube for guiding discarded cuvettes from the cuvette discarding port 111 toward the top of the discarding box may be provided.

The coagulation table 107 and the sensor for measuring the contents of the cuvette herein set, and the like, and the LPIA table 108 and a sensor for measuring the contents of the cuvette herein set, and the like are examples of the measurement unit for performing prescribed measurements, respectively. In the present invention, one is referred to as a "first measurement unit", and the other is referred to as a "second measurement unit". Further, the cuvette chuck unit 109 is also referred to as a "transport unit".

### <Effects>

In accordance with the present embodiment, when a composite analyzer including a plurality of measurement units carries out random measurement for which the order of the examination items to be measured can be freely specified, the movement of the cuvette chuck unit 109 or the reagent nozzle unit 106 is made a simple linear motion, which can reduce the size of the transferring mechanism, and can suppress the cost of the whole device, and can achieve power saving. Further, the rail 110 can be shared between the cuvette chuck unit 109 and the reagent nozzle unit 106, which can suppress an increase in size of the whole device. Further, it becomes possible to efficiently use the prescribed measuring time and space, and it is possible to attain a high measurement processing capability.

### <Analysis processing>

For example, when the latex coagulation measurement is carried out, the LPIA table 108 rotates, and the prescribed holding hole moves to the attachment and detachment position, and stops. Further, the cuvette chuck unit 109 grips one cuvette from the cuvette supply port 1021, and moves the cuvette to the holding hole situated at the attachment and detachment position of the LPIA table 108, and allows the holding hole to hold the cuvette. Subsequently, the holding hole holding the cuvette moves to a prescribed dispensing position. Incidentally, the attachment and detachment position and the dispensing position may be the same. Further, the sample rack 1011 is transported so that a desirable blood collection tube is moved to a prescribed collection position. Then, the sample nozzle unit 103 collects a sample from the blood collection tube previously held at the sample rack 1011 at the collection position, and discharges the sample into the cuvette at the dispensing position of the LPIA table 108. Further, the reagent table 104 rotates, and the tip part of the reagent lid opening/closing unit 105 moves at a prescribed timing so as to come in contact with the lid of the reagent bottle, thereby opening the lid of the prescribed reagent bottle, previously held at the reagent table 104. Then, the reagent bottle with the lid opened moves to a prescribed collection position. At this step, further, the reagent nozzle unit 106 collects a reagent at a prescribed collection position, and discharges the reagent into the cuvette at the dispensing position of the LPIA table 108. Subsequently, the reagent table 104 rotates, and the tip part of the reagent lid opening/closing unit 105 closes the lid of the reagent bottle. Incidentally, the reagent lid opening/closing unit 105 can press the lid vertically from upward to downward of the reagent bottle, thereby closing the lid. Further, the LPIA table 108 rotates, and the cuvette moves to a prescribed stirring position. At the stirring position, a stirring rod is inserted into the concave part disposed at the bottom surface of the cuvette from vertically below, and is displaced so that the tip of the stirring rod draws a circle on a horizontal plane, thereby stirring the contents of the cuvette. Incidentally, it is also acceptable that a plurality of reagents are dispensed. When a plurality of reagents are used, a first reagent may be discharged into the cuvette before dispensing a sample, or may be discharged after dispensing a sample. A second reagent may be, for example, discharged into a cuvette after dispensing a sample. Further, the rotation of the LPIA table 108 causes the cuvette to pass through the optical measurement part formed by the light source and the light receptive part. Thus, at the optical measurement part, the change based on the reaction of the contents is measured based on the transmittance and the absorbance for a specific wavelength, a scattered light, and the like. When the prescribed measurement has been completed, the cuvette is removed from the holding hole by the cuvette chuck unit 109 at the attachment and detachment position of the LPIA table 108. Further, the cuvette chuck unit 109 transports the removed cuvette to the cuvette discarding port 111, and disposes of the cuvette. Such processing can be performed in parallel with respect to the plurality of cuvettes held at the LPIA table 108.

Further, for example, when the coagulation time measurement is carried out, the coagulation table 107 slides, the prescribed holding hole moves to the attachment and detachment position, and stops. Whereas, the cuvette chuck unit 109 grips one cuvette from the cuvette supply port 1021, and moves the cuvette to the holding hole situated at the attachment and detachment position of the LPIA table 108, and allows the holding hole to hold the cuvette. Subsequently, the holding hole holding the cuvette moves to a prescribed dispensing position. Incidentally, the attachment and detachment position and the dispensing position may be the same. Further, the sample rack 1011 is transported so that a desirable blood collection tube is moved to a prescribed collection position. Then, the sample nozzle unit 103 collects a sample from the blood collection tube previously held at the sample rack 1011 at the collection position, and discharges the sample into the cuvette at the dispensing position of the LPIA table 108. Then, the holding hole holding the cuvette moves to the attachment and detachment position of the LPIA table 108, and the cuvette is gripped by the cuvette chuck unit 109, and is transferred to the attachment and detachment position of the coagulation table 107. Further, the reagent table 104 rotates, and the tip part of the reagent lid opening/closing unit 105 moves at a prescribed timing so as to come in contact with the lid of the reagent bottle, thereby opening the lid of the prescribed reagent bottle previously held at the reagent table 104. Then, the reagent bottle with the lid opened moves to a prescribed collection position. Further, the reagent nozzle unit 106 collects a reagent at a prescribed collection position, and discharges the reagent into the cuvette at the dispensing position of the coagulation table 107. Subsequently, the reagent table 104 rotates, and the tip part of the reagent lid opening/closing unit 105 closes the lid of the reagent bottle. Incidentally, the reagent lid opening/closing unit 105 can press the lid vertically from upward to downward of the reagent bottle, and can close the lid. Further, the coagulation table 107 slides, and the cuvette moves to a prescribed stirring position. At the stirring position, a stirring rod is inserted into the concave part disposed at the bottom surface of the cuvette from vertically below, and is displaced so that the tip of the stirring rod draws a circle on a horizontal plane, thereby stirring the contents of the cuvette. Incidentally, it is also acceptable that a plurality of reagents are dispensed. When a plurality of reagents are used, a first reagent may be discharged into the cuvette before dispensing a sample, or may be discharged after dispensing a sample. A second reagent may be, for example, discharged into a cuvette after dispensing a sample. Further, each holding hole of the coagulation table 107 is provided with the optical measurement part formed by the light source and the light receptive part so as to sandwich the cuvette. Thus, at the optical measurement part, the change based on the coagulation of the contents is measured based on the transmittance and the absorbance for a specific wavelength, and the like. When the prescribed measurement has been completed, the cuvette is removed from the holding hole by the cuvette chuck unit 109 at the attachment and detachment position of the coagulation table 107. Further, the cuvette chuck unit 109 transports the removed cuvette to the cuvette discarding port 111, and disposes of the cuvette. Such processing can be performed in parallel with respect to the plurality of cuvettes held at the coagulation table 107.

### <Measuring time extension processing>

Fig. 13 is a view showing one example of a block view showing the function of measuring the blood coagulation time with the composite analyzer. The composite analyzer 1000 measures the blood coagulation time using the coagulation table 107. The computer 21 accommodated in the tank, etc.-accommodating part 2 of Fig. 1 is connected to the sensor provided at the coagulation table 107 via a signal line. Then, the computer 21 measures the degree of coagulation of the blood in the cuvette based on, for example, the absorbance of a prescribed frequency band.

As shown in Fig. 13, the computer 21 includes a processor 211 and a storage device 212, and is connected to a sensor provided at the coagulation table 107 via an input/output interface. The coagulation table 107 includes a light source 1073 for emitting a light with a prescribed wavelength, and a light receptive part 1074 for receiving a light which has passed through the cuvette held by the holding hole 1072 of the cuvette, and the light receptive part 1074 outputs a voltage or a current corresponding to the intensity of an incident light.

The processor 211 is, for example, an arithmetic device such as a CPU (Central Processing Unit), and executes processing in accordance with the present embodiment by executing a program. In the example of Fig. 13, a functional block is shown in the processor 211. Namely, the processor 211 functions as a device control part 2111, a data acquisition part 2112, a coagulation determination part 2113, and an extension determination part 2114. The device control part 2111 controls the composite analyzer 1000 so as to perform the analysis designated with respect to the sample based on the operation by a user. The data acquisition part 2112 acquires data outputted by the units included in the composite analyzer 1000 such as the sensors provided at the coagulation table 107, and the like via a prescribed input/output interface. The coagulation determination part 2113 determines the degree of coagulation of the blood in a cuvette based on the absorbance of each cuvette acquired from the light receptive part 1074 of the coagulation table 107. The extension determination part 2114 determines whether the measurement of each cuvette is terminated or extended based on the determination of the coagulation determination part 2113 at a prescribed timing.

The storage device 212 is, for example, a main storage device such as a RAM (Random Access Memory) or a ROM (Read Only Memory), or an auxiliary storage device such as a HDD (Hard-disk Drive), a SSD (Solid State Drive), an eMMC (embedded Multi-Media Card), or a flash memory. The main storage device ensures the work area of the processor 211, and temporarily stores the data outputted by the sensor, and the like. Further, the auxiliary storage device stores the program in accordance with the present embodiment and the data outputted by the sensor, and other data.

Fig. 14 is a processing flowchart showing one example of the measuring time extension processing. The data acquisition part 2112 of the composite analyzer 1000 acquires information indicative of the absorbance for each cuvette held by the coagulation table 107, and allows the storage device 212 to store the information (Fig. 14: S1). Further, the coagulation determination part 2113 and the extension determination part 2114 determine whether a prescribed measuring time has elapsed, and the specimen in the cuvette has been coagulated or not (S2). In the present embodiment, for example, the prescribed measuring time is set at 210 seconds. Further, whether the specimen has been coagulated or not is determined based on the magnitude relation between the absorbance and the prescribed threshold value. When it is determined that a prescribed measuring time has not elapsed, or that the specimen in the cuvette has been coagulated (S2: NO), the coagulation determination part 2113 and the extension determination part 2114 determine that it is the prescribed determination time point and whether the specimen has been coagulated or not (S3). Incidentally, in the present embodiment, for example, the the prescribed determination time points are assumed to be 30 seconds, 60 seconds, 120 seconds, 180 seconds, and 210 seconds. When it is determined that it is not the prescribed determination time point, or that the specimen in the cuvette has not been coagulated (S3: NO), the process returns to the processing of S1, and the absorbance is continuously recorded. On the other hand, when it is determined that it is the prescribed determination time point, and the specimen in the cuvette has been coagulated (S3: YES), the measurement on the cuvette is completed, and the process transmits to the processing of S7. On the other hand, in S2, when it is determined that the prescribed measuring time has elapsed, and the specimen in the cuvette has not been coagulated (S2: YES), the measuring time is extended (S4), and the process transfers to the reanalysis mode. In the reanalysis mode, the measurement completion time is extended to 360 seconds, and the measurement from 210 seconds onwards is continuously performed (S5). Subsequently, the data acquisition part 2112 determines whether the measurement is completed, or not (S6). In the present step, whether 360 seconds of the measurement completion time after extension has elapsed from the start of the measurement is determined. When it is determined that the measurement completion time has not elapsed (S6: NO), the process returns to the processing of S5, and the measurement is continued. On the other hand, when it is determined that the measurement completion time has elapsed in S6 (S6: YES), or it is determined that it has been the determination time point, and coagulation has been achieved in S3 (S3: YES), the device control part 2111 causes the cuvette to be discarded (S7). In the present step, the cuvette which has completely undergone the measurement is moved to the attachment and detachment position by sliding the coagulation table 107, and the cuvette is gripped by the cuvette chuck unit 109, and is transferred to the cuvette discarding port 111 for being dropped.

The time required for blood to be coagulated is about 10 to 12 seconds for PT, and about 25 to 40 seconds for APTT in the case of a normal specimen. In the measuring time extension processing, the schedule for exchange of cuvettes to be mounted on the measurement unit is determined based on the measuring time (e.g., 210 seconds) in which generally the prescribed reaction is completed. In the case where the measurement is not completed even when the elapsed time exceeds the time serving as the standard, the measuring time is extended to continue the measurement processing. With this procedure, the measuring time of one specimen is not required to be set longer to be safe, which can suppress the reduction of the processing efficiency. Further, when the measurement is once terminated in the measuring time serving as the standard, and the prescribed reaction has not been completed, the measurement is redone for the specimen. Such a processing is inferior in efficiency in that time is taken for redoing and in that the specimen which has not completely undergone the reaction is wasted. By adopting the processing in which the measurement is continued when the reaction has not been completed even after an elapse of the initial measurement completion time, it is possible to eliminate the waste of time and the waste of the specimen in the present embodiment, leading to the improvement of the processing efficiency. Such extension processing is applicable to the processing of determining the degree of progress of some reaction, not limited to coagulation of blood. Further, when it is determined that the reaction has been completed at 30 seconds, 60 seconds, 120 seconds, 180 seconds, or 210 seconds of the prescribed determination time point prior to the measurement completion time, for example, the storage device 212 may store the results, or the monitor 3 may output the results. With this procedure, a user can know the analysis results quickly.

### <Crossmixing test>

Fig. 15 is a view showing one example of a block view indicating the function of supporting the execution of a crossmixing test with the composite analyzer. The composite analyzer 1000 prepares a prescribed specimen in a sample cup 1013 using the sample nozzle unit 103. Further, using the coagulation table 107, the coagulation time of a prescribed specimen is measured. Further, the computer 21 accommodated in the tank, etc.-accommodating part 2 of Fig. 1 is connected to the sensor disposed at the coagulation table 107 via a signal line. Then, the computer 21 measures the degree of coagulation of blood in the cuvette based on, for example, the absorbance within a prescribed frequency band.

The configuration of the computer 21 is substantially the same as that of the example of Fig. 13. In Fig. 15, the configuration corresponding to that of Fig. 13 is given the same reference numeral and sign, and a description thereon is omitted. Also in Fig. 15, a functional block is shown in the processor 211. Namely, the processor 211 functions as the device control part 2111, the data acquisition part 2112, and the output control part 2115. The device control part 2111 controls the composite analyzer 1000 so as to cause the sample cup 1013 to prepare a prescribed specimen using a sample, and to perform the analysis designated for the prepared specimen based on the operation by a user. The data acquisition part 2112 acquires the data outputted by the units included in the composite analyzer 1000 such as the sensors provided at the coagulation table 107, and the like via a prescribed input/output interface. The output control part 2115 draws, for example, a table or a graph using the acquired data, and causes the monitor 3, and the like to output the results.

Fig. 16 is a processing flowchart showing one example of the crossmixing test support processing. The device control part 2111 of the composite analyzer 1000 collects samples from the blood collection tube 1012 holding the sample of a patient and the blood collection tube 1012 holding the normal sample of an able-bodied person, respectively, using the sample nozzle unit 103, and prepares a specimen of a mixture thereof at a prescribed ratio in the sample cup 1013 (S11). In the present step, mixing is performed at a plurality of ratios adopted in a general crossmixing test, so that a mixed specimen is prepared. Specifically, mixed specimens of the samples of a patient and the normal samples at a ratio of 9 to 1, 8 to 2, 5 to 5, and 2 to 8 are formed. Incidentally, in the crossmixing test, the specimens of 10 to 0, 9 to 1, 8 to 2, 5 to 5, 2 to 8, and 0 to 10 are used. Further, in the present step, the specimens may only be prepared in an amount enabling the measurement of a plurality of specimens. For example, the specimens are prepared in an amount necessary for use in the coagulation time measurement of the immediate reaction and the delayed reaction. For example, mention may be made of the following case: when one coagulation time measurement of the immediate reaction and one coagulation time measurement of the delayed reaction are performed, specimens in an amount for at least two measurements (two specimens) are prepared. Then, the specimens are used for the "immediate reaction" for which the coagulation time is measured immediately after mixing, and the "delayed reaction" for which the coagulation time is measured after incubating the mixed specimen at 37 degrees for 2 hours. In the present invention, as the measurement of the coagulation time, mention may be made of the measurements of PT (Prothrombin Time) and/or APTT (Activated Partial Thromboplastin Time).

Further, the data acquisition part 2112 measures the coagulation time of the immediate reaction (S12). In the present step, the mixed specimen held by the sample cup 1013 for the immediate reaction is dispensed into a cuvette at the LPIA table 108, and is transferred to the coagulation table 107, for measuring the coagulation time. Incidentally, with the measurement of the coagulation time, the measuring time extension processing may be performed.

Further, for the mixed specimen for the delayed reaction, using another device by a user, or by a temperature adjusting device not shown included in the composite analyzer 1000, heating is performed, for example, at 37 degrees for two hours. Further, when the sample cup 1013 holding the mixed sample after heating is mounted on the transport space 101, the device control part 2111 transports the sample cup 1013 so as to move to the collection position of the sample nozzle unit 103 (S13). Further, in the present step, in response to the input of the identifying information of the same mixed sample as that used for the immediate reaction, corresponding to the mixed sample after heating, linking of the identifying information is performed based on the control by a user. The rest obtained by removing the specimens used for the immediate reaction from the mixed specimens prepared in an amount enabling the measurement of a plurality of specimens in the sample cup is used as the specimen for the delayed reaction. As a result, the dead volume necessary for dispensing into the cuvette can be reduced, so that the amount of the specimen to be used may be small.

Further, the data acquisition part 2112 measures the coagulation time of the delayed reaction (S14). In the present step, the mixed specimen held by the sample cup 1013 for the immediate reaction is dispensed into a cuvette at the LPIA table 108, and is transferred to the coagulation table 107 for measuring the coagulation time. Also in the present step, with the measurement of the coagulation time, the measuring time extension processing may be performed.

Then, the output control part 2115 outputs the coagulation times of the immediate reaction and the delayed reaction (S15). In the present step, a graph or a table may be drawn, to be outputted to the monitor 3, another computer, the storage device 212, or the like.

Fig. 17 is a view showing one example of the measurement results to be outputted. With the graph of Fig. 17, the vertical axis indicates the coagulation time, and the horizontal axis indicates the mixing ratio of the patient blood plasma and the normal blood plasma. Further, the line graph of a solid line is formed by plotting the coagulation times of the immediate reaction, and the line graph of a broken line is formed by plotting the coagulation times of the delayed reaction. In S15, such a graph may be outputted, or the table indicating the contents may be outputted. Further, the data may be outputted to the storage device 212 in a file form for handling the contents in a CSV (Comma-Separated Values) or a prescribed spreadsheet program, or the like.

With the crossmixing test support processing, the mixed specimens in an amount required for the measurements of the immediate reaction and the delayed reaction is automatically prepared. The specimens may be prepared in an amount enabling the measurements of a plurality of specimens. For example, specimens are prepared in an amount required for use in the coagulation time measurements of the immediate reaction and the delayed reaction. For example, mention may be made of the following case: when one coagulation time measurement of the immediate reaction and one coagulation time measurement of the delayed reaction are performed, specimens in an amount for at least two measurements (two specimens) are prepared. Therefore, it does not take time and effort of a user to prepare the specimens for use in the measurements of the immediate reaction and the delayed reaction. Further, the specimens to be respectively used for the immediate reaction and the delayed reaction can be prepared altogether. This eliminates the fear that an error is caused in formation of the specimens. Further, the coagulation times in the immediate reaction and the delayed reaction can be measured with the composite analyzer 1000. For this reason, for example, as shown in Fig. 17, the results can be outputted in a form easy for a user to make comparison therebetween, which is useful.

Incidentally, the mixing pattern is not limited to 10 to 0, 9 to 1, 8 to 2, 5 to 5, 2 to 8, and 0 to 10. For example, 7 points of 1 to 0, 9 to 1, 3 to 1, 1 to 1, 1 to 3, 1 to 9, and 0 to 1 are acceptable. Alternatively, 5 points of 1 to 0, 3 to 1, 1 to 1, 1 to 3, and 0 to 1 are also acceptable. Still alternatively, 3 points of 1 to 0, 1 to 1, and 0 to 1 are also acceptable. Even these patterns can indicate whether the graph is convex upward or convex downward, and provides a hint to identification of the cause of the extension of the coagulation time. By previously setting the patterns, and making the patterns selectable, a user can give an order of the measurement with ease. Further, by automatically calculating and displaying the specimen amount required for each pattern, a user can select the pattern according to the remaining amount of the specimen.

### <Modified example>

The embodiments and the modified examples are examples, and the present invention is not limited to the configuration.

Fig. 18 is a schematic view showing one example of the composite analyzer 1000 including three measurement units. The number of the measurement units is not limited to two. In the example of Fig. 18, a third measurement unit 112 is added to the left side of the LPIA table 108 in a plan view. When three or more measurement units are included, the cuvette chuck unit 109 and the reagent nozzle unit 106 move linearly along the rail 110, and the three measurement units are arranged on a straight line roughly in parallel with the rail. With this configuration, the movements of the cuvette chuck unit 109 and the reagent nozzle unit 106 can be made simple linear motions, resulting in power saving. Further, the rail 110 can be shared between the cuvette chuck unit 109 and the reagent nozzle unit 106, so that the whole device can be reduced in size.

Further, the present disclosure includes the method and the computer program for executing the processing, and a computer readable recording medium recording the program. The recording medium recording the program thereon causes the computer to execute the program, which enables the processing.

Herein, the computer readable recording medium denotes a recording medium capable of storing information such as data and program by electric, magnetic, optical, mechanical, or chemical action, which can be read by a computer. Of such recording media, those removable from the computer include a flexible disk, a magneto-optical disk, an optical disk, a magnetic disk, a memory card, and the like. Further, the recording media fixed to a computer include a HDD, a SSD (Solid State Drive), a ROM, and the like.

### REFERENCE SIGNS LIST

- 1000: Composite analyzer
- 1: Measurement unit accommodation part
- 101: Transport space
- 1011: Sample rack
- 1012: Sample container
- 102: Cuvette supply unit
- 1021: Cuvette supply port
- 1022: Hopper
- 103: Sample nozzle unit
- 1031: Dispensing position
- 1032: Nozzle washing tank
- 1033: Rotation shaft
- 1034: Nozzle
- 104: Reagent table
- 1041: Collection position
- 1042: Setting part
- 1043: Convex part
- 105: Reagent lid opening/closing unit
- 1051: Tip part
- 106: Reagent nozzle unit
- 1061: Nozzle washing tank
- 1062: Nozzle
- 107: Coagulation table
- 1071: Attachment and detachment position
- 1072: Holding hole
- 1073: Light source
- 1074: Light receptive part
- 1075: Driving part
- 1076: Dispensing position
- 108: LPIA table
- 1081: Attachment and detachment position
- 1082: Dispensing position
- 1083: Rotation shaft
- 1084: Holding hole
- 1085: Spring
- 109: Cuvette chuck unit
- 1091: Two-finger gripper
- 110: Rail
- 111: Cuvette discarding port
- 112: Third measurement unit
- 2: Tank, etc.-accommodating part
- 3: Monitor
- 4: Status output part

## Claims

1. An analyzer comprising:
a sample rack (1011) for holding a plurality of blood collection tubes (1012), wherein each blood collection tube accommodates a biological sample;
a mechanism for transporting the sample rack (1011) such that a desirable blood collection tube (1012) of the plurality of blood collection tubes (1012) is arranged at a prescribed sample collection position,
a sample nozzle unit (103) for collecting a biological sample from the desired blood collection tube (1012) held in the sample rack (1011) and discharging said sample to a cuvette held at a prescribed sample dispensing position (1031) ;
a first measurement unit (107) for holding the cuvette to which the biological sample is dispensed, and performing a first measurement of a content of the cuvette;
a second measurement unit (108) for holding the cuvette, and performing a second measurement different from the first measurement of the content of the cuvette;
a transport unit (109) for gripping the cuvette, and transporting the cuvette to a first attachment and detachment position (1071) of the first measurement unit (107) or a second attachment and detachment position (1081) of the second measurement unit (108);
a reagent table (104) for holding a plurality of reagent containers for holding reagents; and
a reagent nozzle unit (106) for collecting the reagent from the reagent container at a prescribed collection position (1041), and dispensing the collected reagent in the cuvette held by the first measurement unit (107) at a first prescribed dispensing position (1076) or the second measurement unit (108) at a second prescribed dispensing position (1082),
wherein the transport unit (109) is connected to a guide rail (110) and moves in a horizontal direction along the guide rail (110), and in a vertical direction,
wherein movement of the transport unit (109) in a forward/backward direction is prevented,
wherein the forward/backward direction is horizontal and perpendicular to the guide rail (110),
wherein the first attachment and detachment position (1071) of the first measurement unit (107) and the second attachment and detachment position (1081) of the second measurement unit (108) are provided on a first line substantially along the guide rail in a plan view,
wherein the reagent nozzle unit (106) is connected to the guide rail (110) and moves in the horizontal direction along the guide rail (110),
wherein the collection position (1041) of the reagent table (104) and the first and second prescribed dispensing positions (1076, 1082) of the first (107) and second (108) measurement units are each provided on a second line substantially in parallel with the guide rail (110),
wherein the sample nozzle unit (103) includes a sample nozzle (1034) that moves in a prescribed movable range that is different from a movable range of the transport unit (109) along the guide rail and that is different from a moveable range of the reagent nozzle unit (106) along the guide rail,
wherein the second measurement unit (108) is configured to move the cuvette to the prescribed sample dispensing position (1031), and
wherein the sample collection position and the sample dispensing position (1031) are provided on the prescribed movable range of the sample nozzle (103).

2. The analyzer according to claim 1, further comprising a supply unit (102) for supplying the cuvette from a supply port (1021) thereof to the transport unit (109),
wherein the supply port (1021) of the supply unit (102), the first attachment and detachment position (1071) of the first measurement unit (107), and the second attachment and detachment position (1081) of the second measurement unit (108) are arranged on the first line.

3. The analyzer according to claim 1 or 2, further comprising a discarding port (111) for discarding the cuvette,
wherein the discarding port (111), the first attachment and detachment position (1071) of the first measurement unit (107), and the second attachment and detachment position (1081) of the second measurement unit (108) are arranged on the first line.

4. The analyzer according to any one of claims 1-3, further comprising a reagent nozzle washing tank (1061) for washing a reagent nozzle arranged at the reagent nozzle unit (106),
wherein the reagent nozzle washing tank (1061) is arranged on the second line on which the collection position (1041) of the reagent table (104) and the first and second prescribed dispensing positions (1078, 1082) of the first (107) and second (108) measurement units are arranged.

5. The analyzer according to any one of claims 1 to 4,
wherein the guide rail (110) is provided substantially linearly.

6. The analyzer according to any one of claims 1 to 5,
wherein the first measurement unit (107) outputs data indicative of a degree of progress of a coagulation reaction effected in blood accommodated in the cuvette, and
wherein the analyzer further comprises a processor for extending a measuring time and continuing measurement processing when determination is made that, even after an elapse of a preset measuring time, the prescribed reaction has not been completed on the basis of the data outputted from the first measurement unit (107).

## Patentansprüche

1. Analysevorrichtung, umfassend:
einen Probenständer (1011) zum Halten einer Mehrzahl von Blutentnahmeröhrchen (1012), wobei jedes Blutentnahmeröhrchen eine biologische Probe aufnimmt;
einen Mechanismus zum Transportieren des Probenständers (1011) derart, dass ein erwünschtes Blutentnahmeröhrchen (1012) der Mehrzahl von Blutentnahmeröhrchen (1012) an einer vorgeschriebenen Probenentnahmeposition angeordnet wird,
eine Probenentnahmedüsen-Einheit (103) zur Entnahme einer biologischen Probe aus dem gewünschten, im Probenständer gehaltenen Blutentnahmeröhrchen (1012) und zur Abgabe der Probe an eine Küvette an einer vorgeschriebenen Probenabgabeposition (1031);
eine erste Messeinheit (107) zum Halten der Küvette, an die die biologische Probe abgegeben wird, und zum Durchführen einer ersten Messung eines Inhalts der Küvette;
eine zweite Messeinheit (108) zum Halten der Küvette und zum Durchführen einer sich von der ersten Messung unterscheidenden zweiten Messung des Inhalts der Küvette;
eine Transporteinheit (109) zum Greifen der Küvette und zum Transportieren der Küvette zu einer ersten Position (1071) zum Anbringen und Abnehmen der ersten Messeinheit (107) oder zu einer zweiten Position (1081) zum Anbringen und Abnehmen der zweiten Messeinheit (108);
einen Reagenztisch (104) zum Halten einer Mehrzahl von Reagenzbehältern zur Aufnahme von Reagenzien; und
eine Reagenzdüsen-Einheit (106) zur Entnahme des Reagens aus dem Reagenzbehälter an einer vorgeschriebenen Entnahmeposition (1041) und zur Abgabe des entnommenen Reagens an die Küvette, die durch die erste Messeinheit (107) an einer ersten vorgeschriebenen Abgabeposition (1076) oder durch die zweite Messeinheit (108) an einer zweiten vorgeschriebenen Abgabeposition (1082) gehalten wird,
wobei die Transporteinheit (109) mit einer Führungsschiene (110) verbunden ist und sich in einer horizontalen Richtung entlang der Führungsschiene (110) und sich in einer vertikalen Richtung bewegt,
wobei eine Bewegung der Transporteinheit (109) in einer Vorwärts-/Rückwärtsrichtung verhindert wird,
wobei die Vorwärts-/Rückwärtsrichtung horizontal und senkrecht zur Führungsschiene (110) ist,
wobei die erste Position (1071) zum Anbringen und Abnehmen der ersten Messeinheit (1078) und die zweite Position (1081) zum Anbringen und Abnehmen der zweiten Messeinheit (108) in der Draufsicht an einer ersten Linie im Wesentlichen entlang der Führungsschiene vorgesehen sind,
wobei die Reagenzdüsen-Einheit (106) mit der Führungsschiene (110) verbunden ist und sich in der horizontalen Richtung entlang der Führungsschiene (110) bewegt,
wobei die Entnahmeposition (1041) des Reagenztisches (104) und die erste und zweite vorgeschriebene Abgabeposition (1076, 1082) der ersten (107) und zweiten (108) Messeinheit jeweils an einer zweiten Linie im Wesentlichen parallel zur Führungsschiene (110) vorgesehen sind,
wobei die Probendüsen-Einheit (103) eine Probendüse (1034) aufweist, die sich in einem vorgeschriebenen Bewegungsbereich bewegt, der sich von einem Bewegungsbereich der Transporteinheit (109) entlang der Führungsschiene unterscheidet und der sich von einem Bewegungsbereich der Reagenzdüsen-Einheit (106) entlang der Führungsschiene unterscheidet,
wobei die zweite Messeinheit (108) dafür eingerichtet ist, die Küvette zur vorgeschriebenen Probenabgabeposition (1031) zu bewegen, und
wobei die Probenentnahmeposition und die Probenabgabeposition (1031) an dem vorgeschriebenen Bewegungsbereich der Probendüse (103) vorgesehen sind.

2. Analysevorrichtung nach Anspruch 1, ferner umfassend eine Zuführeinheit (102) zum Zuführen der Küvette von einem Zuführport (1021) derselben zur Transporteinheit (109),
wobei der Zuführport (1021) der Zuführeinheit (102), die erste Position (1071) zum Anbringen und Abnehmen der ersten Messeinheit (107) und die zweite Position (1081) zum Anbringen und Abnehmen der zweiten Messeinheit (108) an der ersten Linie angeordnet sind.

3. Analysevorrichtung nach Anspruch 1 oder 2, ferner umfassend einen Entsorgungs-Port (111) zum Entsorgen der Küvette,
wobei der Entsorgungs-Port (111), die erste Position (1071) zum Anbringen und Abnehmen der ersten Messeinheit (107) und die zweite Position (1081) zum Anbringen und Abnehmen der zweiten Messeinheit (108) an der ersten Linie angeordnet sind.

4. Analysevorrichtung nach einem der Ansprüche 1-3, ferner umfassend einen Reagenzdüsen-Waschbehälter (1061) zum Waschen einer Reagenzdüse, die an der Reagenzdüseneinheit (106) angeordnet ist,
wobei der Reagenzdüsen-Waschbehälter (1061) an der zweiten Linie angeordnet ist, an der die Entnahmeposition (1041) des Reagenztisches (104) und die erste und zweite vorgeschriebene Abgabeposition (1078, 1082) der ersten (107) und zweiten (108) Messeinheit angeordnet sind.

5. Analysevorrichtung nach einem der Ansprüche 1 bis 4,
wobei die Führungsschiene (110) im Wesentlichen linear vorgesehen ist.

6. Analysevorrichtung nach einem der Ansprüche 1 bis 5,
wobei die erste Messeinheit (107) Daten ausgibt, die auf einen Grad des Fortschreitens einer Blutgerinnungsreaktion von Blut in der Küvette schließen lassen, und
wobei das Analysegerät ferner einen Prozessor umfasst zur Messzeitverlängerung und zum Fortsetzen der Messdatenverarbeitung, wenn auf der Basis der von der ersten Messeinheit (107) ausgegebenen Daten festgestellt wird, dass selbst nach Verstreichen einer voreingestellten Messdauer die vorgeschriebene Reaktion noch nicht abgeschlossen ist.

## Revendications

1. Dispositif d'analyse comprenant:
un porte-échantillons (1011) destiné à maintenir une pluralité de tubes de prélèvement sanguin (1012), chaque tube de prélèvement sanguin contenant un échantillon biologique;
un mécanisme destiné à déplacer le porte-échantillons (1011) de telle sorte qu'un tube de prélèvement sanguin (1012) souhaité parmi la pluralité de tubes de prélèvement sanguin (1012) soit positionné à une position de prélèvement d'échantillon prescrite,
une unité de buse de prélèvement d'échantillon (103) destinée à prélever un échantillon biologique à partir du tube de prélèvement sanguin souhaité (1012) maintenu dans le porte-échantillons et à décharger l'échantillon vers une cuvette à une position de distribution d'échantillon prescrite (1031);
une première unité de mesure (107) destinée à maintenir la cuvette dans laquelle l'échantillon biologique est distribué et à effectuer une première mesure du contenu de la cuvette;
une deuxième unité de mesure (108) destinée à maintenir la cuvette et à effectuer une deuxième mesure du contenu de la cuvette, différente de la première mesure;
une unité de transport (109) destinée à saisir la cuvette et à la transporter vers une première position de fixation et de détachement (1071) de la première unité de mesure (107), ou vers une deuxième position de fixation et de détachement (1081) de la deuxième unité de mesure (108);
une table de réactifs (104) destinée à supporter une pluralité de récipients pour réactifs destinés à contenir des réactifs; et
une unité de buses à réactifs (106) destinée à prélever le réactif du récipient pour réactifs à une position de prélèvement prescrite (1041) et à distribuer le réactif prélevé dans la cuvette maintenue par la première unité de mesure (107) à une première position de distribution prescrite (1076) ou par la deuxième unité de mesure (108) à une deuxième position de distribution prescrite (1082),
l'unité de transport (109) étant reliée à un rail de guidage (110) et se déplaçant dans une direction horizontale le long du rail de guidage (110) ainsi que dans une direction verticale,
le mouvement de l'unité de transport (109) dans une direction avant/arrière étant empêché,
la direction avant/arrière étant horizontale et perpendiculaire au rail de guidage (110),
la première position de fixation et de détachement (1071) de la première unité de mesure (1078) et la deuxième position de fixation et de détachement (1081) de la deuxième unité de mesure (108) étant prévues, en vue de dessus, sur une première ligne s'étendant sensiblement le long du rail de guidage,
l'unité de buses de réactifs (106) étant reliée au rail de guidage (110) et se déplaçant dans la direction horizontale le long du rail de guidage (110),
la position de prélèvement (1041) de la table de réactifs (104) ainsi que les première et deuxième positions de distribution prescrites (1076, 1082) des première (107) et deuxième (108) unités de mesure étant respectivement prévues sur une deuxième ligne sensiblement parallèle au rail de guidage (110),
l'unité de buse de prélèvement d'échantillon (103) comportant une buse d'échantillonnage (1034) qui se déplace dans une plage de déplacement prescrite, distincte de la plage de déplacement de l'unité de transport (109) le long du rail de guidage et distincte de la plage de déplacement de l'unité de buse de réactifs (106) le long du rail de guidage,
la deuxième unité de mesure (108) étant agencée pour déplacer la cuvette vers la position de distribution d'échantillon prescrite (1031), et
la position de prélèvement d'échantillon et la position de distribution d'échantillon (1031) étant prévues dans la plage de déplacement prescrite de la buse de prélèvement d'échantillon (103).

2. Dispositif d'analyse selon la revendication 1, comprenant en outre une unité d'acheminement (102) destinée à acheminer la cuvette depuis un orifice d'acheminement (1021) de celle-ci vers l'unité de transport (109),
l'orifice d'acheminement (1021) de l'unité d'acheminement (102), la première position de fixation et de détachement (1071) de la première unité de mesure (107) et la deuxième position de fixation et de détachement (1081) de la deuxième unité de mesure (108) étant disposées sur la première ligne.

3. Dispositif d'analyse selon la revendication 1 ou 2, comprenant en outre un orifice d'évacuation (111) destiné à évacuer la cuvette
ledit orifice d'évacuation (111), la première position de fixation et de détachement (1071) de la première unité de mesure (107) et la deuxième position de fixation et de détachement (1081) de la deuxième unité de mesure (108) étant disposés sur la première ligne.

4. Dispositif d'analyse selon l'une quelconque des revendications 1 à 3, comprenant en outre un réservoir de lavage de buse de réactifs (1061) destiné à laver une buse de réactifs disposée sur l'unité de buses de réactifs (106),
le réservoir de lavage de buse de réactifs (1061) étant disposé sur la deuxième ligne, sur laquelle se trouvent la position de prélèvement (1041) de la table de réactifs (104) ainsi que les première et deuxième positions de distribution prescrites (1078, 1082) des première (107) et deuxième (108) unités de mesure.

5. Dispositif d'analyse selon l'une quelconque des revendications 1 à 4,
dans lequel le rail de guidage (110) est prévu de manière essentiellement linéaire.

6. Dispositif d'analyse selon l'une quelconque des revendications 1 à 5,
dans lequel la première unité de mesure (107) délivre des données indiquant un degré d'avancement d'une réaction de coagulation du sang dans la cuvette, et
dans lequel le dispositif d'analyse comprend en outre un processeur destiné à prolonger la durée de mesure et à poursuivre le traitement des données de mesure lorsqu'il est constaté, sur la base des données fournies par la première unité de mesure (107), que même après l'écoulement d'une durée de mesure prédéfinie, la réaction prescrite n'est pas encore terminée.
